# EUROPEAN PATENT APPLICATION

(11) **EP 2 133 090 A1**
(43) Date of publication of application: **16.12.2009**
(21) Application number: 08721754.3
(22) Date of filing: 11.03.2008
(51) Int. Cl.: A61K 38/00, A61P 5/06

(54) **GROWTH HORMONE SECRETION STIMULATOR**

(30) Priority: 12.03.2007 JP 2007062372
(71) Applicant: Snow Brand Milk Products Co., Ltd., Sapporo-shi Hokkaido 065-0043 (JP)
(72) Inventor: ONO, Aiko, Kawagoe-shi Saitama 350-1165 (JP); MATSUYAMA, Hiroaki, Kawagoe-shi Saitama 350-1165 (JP); MORITA, Yoshikazu, Kawagoe-shi Saitama 350-1165 (JP); SERIZAWA, Atsushi, Kawagoe-shi Saitama 350-1165 (JP); HIGURASHI, Satoshi, Kawagoe-shi Saitama 350-1165 (JP)
(74) Representative: Wakerley, Helen Rachael
(86) International application number: PCT/JP2008/054338
(87) International publication number: WO 2008/111573

(57) **Abstract**

Provided are: an agent for promoting growth hormone-secretion, containing a milk-derived basic protein fraction as an active ingredient, with the promotion of growth hormone-secretion being mediated by the promotion of secretion of ghrelin having an effect for promoting growth hormone-secretion; and a method of promoting the secretion of ghrelin by using a milk-derived basic protein fraction. The agent for promoting growth hormone-secretion may be ingested on a daily basis and is highly safe even with its ingestion for a long period.

## Description

### TECHNICAL FIELD

The present invention relates to an agent for promoting growth hormone-secretion, containing a milk-derived basic protein fraction as an active ingredient. Further, the present invention relates to an agent for promoting growth hormone-secretion, containing a milk-derived basic protein fraction as an active ingredient, with the promotion of growth hormone-secretion being mediated by the promotion of ghrelin secretion. Still further, the present invention relates to a method of promoting the ghrelin secretion by using a milk-derived basic protein fraction.

### BACKGROUND ART

A growth hormone has an effect associated with growth and an effect for controlling metabolism. As far as the effect associated with growth is concerned, it is said that it enhances the cell division in a target organ, promotes expansion of bone, promotes expansion of bone by substituting epiphyseal cartilage with bone tissue, promotes intake of specific amino acids, and thereby involves development of muscle that promotes the synthesis of proteins. As far as the effect for controlling metabolism is concerned, it is said that it involves; an effect for promoting metabolism which contributes to promoting the metabolism of carbohydrates, proteins, and lipids; a hyperglycemia effect which contributes to maintaining the blood glucose level constant because the growth hormone promotes glycogenolysis in the liver and has an anti-insulin effect (secretion of insulin is suppressed to raise the blood glucose level); a homeostasis effect which contributes to maintaining the calcium concentration and the like constant, thereby maintaining homeostasis in the body; and an effect for promoting the recruitment of body fat which is released in the form of free fatty acids from adipose tissues when the body is in short of energy.

Ghrelin is a new peptide for promoting growth hormone-secretion, and was isolated from the stomach of a rat in 1999. It is mainly secreted from the gastrointestinal tract, is a physiological hormone promoting the growth hormone-secretion from the pituitary gland via blood or the vagus afferent. Ghrelin is made of 28 amino acids, and is a peptide having a structure characteristic of the third serine residue being acylated with a fatty acid (n-octanoate) (see Non-patent Document 1). This new peptide has a powerful activity for promoting growth hormone-secretion. Moreover, the following effects have been revealed: promotion of eating (see Non-patent Document 2); functional control such as control of gastrointestinal motility and gastric acid secretion; and an effect on the energy metabolism system such as fat accumulation and an effect on the circulatory system such as vasodilation and enhancement of cardiac function. It is known that ghrelin secretion is promoted by fasting, a low-amino-acid diet, reduction of growth hormone-secretion, stimulation of sympathetic/parasympathetic nerve or the receptors thereof, and cholecystokinin or gastrin. Recently, they have reported; a therapeutic agent for diabetes (see Patent Document 1); treatment of congestive heart failure (see Patent Documents 2 and 3); and an agent for promoting growth hormone-secretion (see Patent Document 4), in which ghrelin or its derivatives are administered. Those reports involve the the administration of ghrelin itself or its derivatives themselves, and do not involve the promotion of ghrelin secretion. Further, because the growth hormone and ghrelin, which are proteins, are decomposed by gastrointestinal enzymes, the ingestion as they are probably does not lead to the exhibition of the effect.

In order to promote the above-mentioned growth hormone-secretion, it is more desired that an agent for promoting secretion, which promotes the secretion of ghrelin having an effect for promoting growth hormone-secretion, be developed, rather than ingestion of drugs containing a growth hormone or ghrelin Further, it is desired that an agent for promoting growth hormone-secretion having the following characteristics be developed: the agent can be ingested on a daily basis and is highly safe even with its ingestion for a long period.
[Patent Document 1] WO 2004/004772
[Patent Document 2] JP 2005-507949 A
[Patent Document 3] JP 2005-535707 A
[Patent Document 4] JP 2005-82489 A
[Non-patent Document 1] Nature 1999, 402: 656-660
[Non-patent Document 2] Nature 2001, 409: 194-198

### DISCLOSURE OF THE INVENTION

### PROBLEM TO BE SOLVED BY THE INVENTION

An object of the present invention is to obtain a substance having an effect for promoting growth hormone-secretion, which can be ingested on a daily basis and is highly safe even with its ingestion for a long period. Further, another object of the present invention is to obtain an agent for promoting growth hormone-secretion, with the promotion of growth hormone-secretion being mediated by the promotion of ghrelin secretion.

### MEANS FOR SOLVING THE PROBLEM

The inventors of the present invention have been searching a growth hormone secretagogue existing in milk in order to obtain a substance having an effect for promoting growth hormone-secretion. As a result, the inventors have discovered that a basic protein which exists in milk in very small amount has an effect for promoting the secretion of ghrelin, which has the effect for promoting growth hormone-secretion. In addition, the inventors have discovered that the milk-derived basic protein fraction can be used as an active ingredient in an agent for promoting growth hormone-secretion, and then, the present invention has been completed.
That is, the present invention relates to an agent for promoting growth hormone-secretion which contains a milk-derived basic protein fraction as an active ingredient. Further, it relates to an agent for promoting growth hormone-secretion, with the promotion of growth hormone-secretion being mediated by the promotion of ghrelin secretion. Still further, it relates to a method of promoting ghrelin secretion, wherein a milk-derived basic protein fraction is used for the promotion.

### EFFECTS OF THE INVENTION

Secretion of ghrelin may be promoted by the the administration of an agent for promoting growth hormone-secretion of the present invention which contains a milk-derived basic protein fraction as an active ingredient. Thus, it is useful for promotion of eating, functional control such as control of gastrointestinal motility and gastric acid secretion, and treatment and prophylaxis of diseases in the energy metabolism system such as fat accumulation and diseases in the circulatory system such as vasodilation and enhancement of cardiac function.
Further, it is useful for elongation of bone, growth of muscle, and hyperglycemia because the administration of an agent for promoting growth hormone-secretion which contains a milk-derived basic protein fraction as an active ingredient, leads to the promotion of growth hormone-secretion mediated by ghrelin secretion.

### BRIEF DESCRIPTION OF THE DRAWINGS

[Figure 1] This figure illustrates a serum ghrelin concentration in each rat for a basic protein fraction-treated group and a control group.
[Figure 2] This figure illustrates a serum ghrelin concentration in each rat for test groups of a basic protein fraction-treated group and a control group.
[Figure 3] This figure illustrates a plasma growth hormone concentration in each rat for a basic protein fraction-treated group and a control group.

### BEST MODE FOR CARRYING OUT THE INVENTION

An agent for promoting growth hormone-secretion of the present invention is characterized by containing a milk-derived basic protein fraction as an active ingredient. The milk-derived basic protein fraction may be obtained from mammalian milk such as cow's milk, human milk, goat's milk, and sheep's milk.
As methods of obtaining such a milk-derived basic protein fraction, for example, the following methods are known: a method involving bringing milk or a milk-derived material into contact with a cation exchanger for basic proteins to adsorb to the cation exchanger, and eluting the basic protein fraction which has adsorbed to the cation exchanger with an eluent having a pH of more than 5 and an ionic strength of more than 0.5 (JP H05-202098 A); a method of obtaining the fraction using an alginate gel (JP S61-246198 A); a method of obtaining the fraction from milk whey using inorganic porous particles (JP H01-86839 A); and a method of obtaining the fraction from milk using a sulfate ester compound (JP S63-255300 A). In the present invention, a milk-derived basic protein fraction obtained by one of the above-mentioned methods can be used.

The milk-derived basic protein fraction according to the present invention has the following properties.
(1) According to sodium dodecyl sulfate polyacrylamide gel electrophoresis (SDS-PAGE), it consists of several kinds of proteins whose molecular weight range from 3,000 to 80,000.
(2) Ninety five (95) weight % or more of it is proteins, and it contains a small amount of fat, ashes and so on.
(3) The proteins mainly consist of lactoferrin and lactoperoxidase.
(4) As the amino acid composition of the proteins, 15 weight % or more of basic amino acids such as lysine, histidine, and arginine are contained.

When the agent for promoting growth hormone-secretion of the present invention is administered, the milk-derived basic protein fraction which is an active ingredient may be used as is. The milk-derived basic protein fraction may also be used after being formulated into a powder, a granule, a tablet, a capsule, a drink, or the like according to a conventional method. Further, the following way may also be possible. These basic protein fractions may be added as is or after being formulated, to a nutrient, food and drink, or the like, thereby promoting growth hormone-secretion. It should be noted that a material containing the milk-derived basic protein fraction may also be subjected to heat sterilization under usual conditions because the milk-derived basic protein fraction is relatively stable to heat.

### EXAMPLE 1

After a column (a diameter of 5 cm by a height of 30 cm) packed with 400 g of sulfonated CHITO PEARL (manufactured by FUJIBO Co., Ltd.), which is a cation exchange resin, was sufficiently washed with deionized water, 40 L of skim milk (pH 6.7) were passed through the column at a flow rate of 25 ml/min. After passing through the liquid, the column was sufficiently washed with deionized water, and a basic protein fraction which adsorbed to the resin was eluted with a 0.02 M carbonate buffer (pH 7.0) containing 0.98 M sodium chloride. Then, the eluate was subjected to desalting with a reverse osmotic (RO) membrane, and the resultant was concentrated, followed by freeze-drying, to thereby yield 21 g of a milk-derived basic protein fraction-powder which is an active ingredient of an agent for promoting growth hormone-secretion of the present invention.

### [Test Example 1]

Measurement was carried out for the basic protein fraction obtained in Example 1, by using sodium dodecyl sulfate polyacrylamide gel electrophoresis (SDS-PAGE). The results showed that the molecular weights of the basic protein fraction were distributed in the range from 3,000 to 80,000.

### [Test Example 2]

The milk-derived basic protein fraction obtained in Example 1 was analyzed for its ingredient composition.
The results are shown in Table 1. As shown in the table, almost all the contents of the fraction are proteins.

**[Table 1]**

| | |
|---|---|
| Water | 1.06 (Weight %) |
| Protein | 96.5 |
| Fat | 0.56 |
| Ash | 0.27 |
| Others | 1.61 |

### [Test Example 3]

The milk-derived basic protein fraction obtained in Example 1 was subjected to hydrolysis with 6 N hydrochloric acid at 110°C for 24 hours, and the resultant was then analyzed for its amino acid composition using an amino acid analyzer (L-8500 type manufactured by Hitachi, Ltd.). Table 2 shows the results. The milk-derived basic protein fraction includes 15 weight % or more of basic amino acids in its amino acid composition.

**[Table 2]**

| | |
|---|---|
| Aspartic acid | 10.1 (weight %) |
| Serine | 5.3 |
| Glutamic acid | 12.3 |
| Proline | 4.7 |
| Alanine | 5.7 |
| Leucine | 10.2 |
| Lysine | 8.4 |
| Histidine | 2.6 |
| Arginine | 7.2 |
| Others | 33.5 |

### EXAMPLE 2

After a column (a diameter of 100 cm by a height of 10 cm) packed with 30 kg of SP-TOYOPEARL (manufactured by TOSOH CORPORATION), which is a cation exchange resin, was sufficiently washed with deionized water, 3 t of cheese whey (pH 6.2), which had been subjected to heat sterilization at 121°C for 30 seconds, were passed through the column at a flow rate of 10 L/min. After the passing through of the liquid, the column was sufficiently washed with deionized water, and a basic protein fraction which adsorbed to the resin was eluted with a 0.1 M citrate buffer (pH 5.7)containing 0.9 M sodium chloride. Then, the eluate was subjected to desalting by a electrodialysis (ED) method, and the resultant was concentrated, followed by freeze-drying, to thereby obtain 183 g of a milk-derived basic protein fraction-powder which is an active ingredient of an agent for promoting hormone-secretion of the present invention.

[Test Example 4] The milk-derived basic protein fraction-powder obtained in Example 1 was subjected to an animal experiment to investigate the ghrelin secretion-promoting effect. Three-week-old Fisher female rats were used for the animal experiment. Those rats were divided into two test groups each including 10 rats, a control group (A group) and a 1 weight % basic protein fraction-treated group (B group), and then, those rats in each group were bred for 2 weeks with test feeds shown in Table 3.

**[Table 3]**

| | Control group (A group) | Treated group (B group) |
|---|---|---|
| Ovalbumin | 15.0 | 14.0 (weight %) |
| Corn starch | 57.21 | 57.21 |
| Cellulose | 5.0 | 5.0 |
| Corn oil | 7.0 | 7.0 |
| Vitamin mixture | 1.0 | 1.0 |
| Mineral mixture (Ca free) | 3.5 | 3.5 |
| CaHPO₄ · H2O | 1.29 | 1.29 |
| Sucrose | 10.0 | 10.0 |
| Basic protein fraction-powder | - | 1.0 |

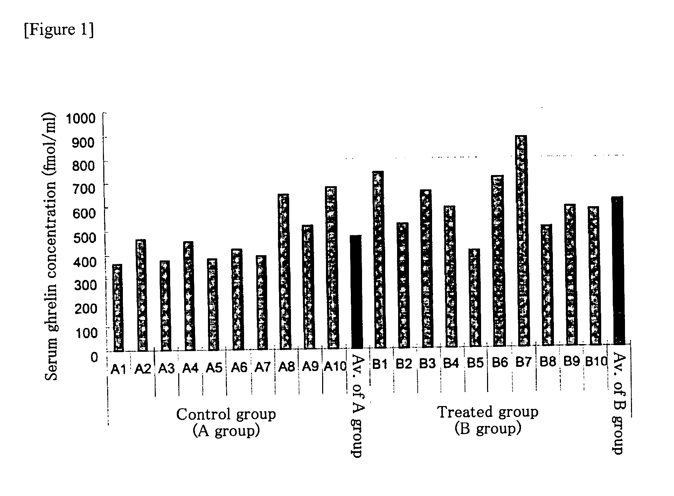

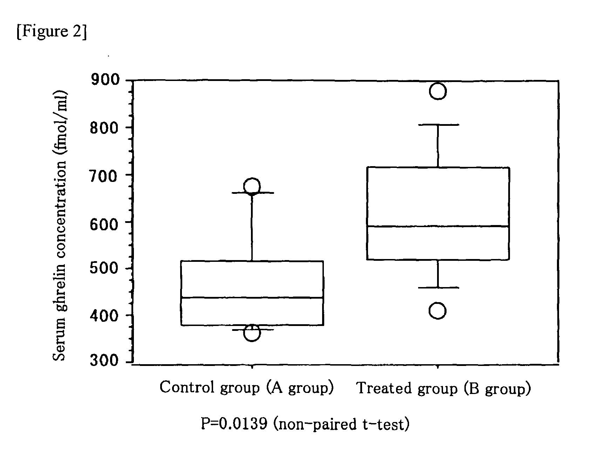

Two weeks after the breeding, blood of the rats in each test group was collected from the abdominal cava of the rats, and serum was immediately separated and refrigerated at -80°C. After that, a ghrelin ELISA kit (SCETI #97752) was used to determine the serum ghrelin concentration. Figures 1 and 2 show the concentration of ghrelin in the rat serum of each test group. As shown in the figures, the serum ghrelin concentration of the group treated with the milk-derived basic protein fraction of the present invention (B group) was significantly high compared with that of the control group (A group).
The above results have clarified that the milk-derived basic protein fraction has an effect for increasing the serum ghrelin concentration, and promotes the secretion of ghrelin having an effect for promoting growth hormone-secretion. Further, it has also been clarified that the ghrelin secretion-promoting effect is observed when 900 mg of the basic protein fraction are administered as the maximum amount in terms of 1 kg of rat body weight.

### EXAMPLE 3

After a column (a diameter of 10 cm by a height of 7.5 cm) packed with 400 g of Macro-Prep high S (manufactured by BioRad Laboratories), which is a cation exchange resin, was sufficiently washed with deionized water, 40 L of skim milk (pH 6.7), which had been subjected to sterilization at 80°C for 15 seconds, were passed through the column at a flow rate of 250 ml/min. After the passing through the liquid, the column was sufficiently washed with deionized water, and a basic protein fraction which adsorbed to the resin was eluted with a 0.02 M carbonate buffer (pH 7.0)containing 1.48 M sodium chloride. Then, the eluate was subjected to desalting with a reverse osmotic (RO) membrane, and the resultant was concentrated, followed by freeze-drying, to thereby yield 25 g of a milk-derived basic protein fraction-powder which is an active ingredient of an agent for promoting growth hormone-secretion of the present invention.

### [Test Example 5]

The milk-derived basic protein fraction-powder obtained in Example 3 was subjected to investigation of the ability for promoting growth hormone-secretion. Seven days after breeding 10-week-old male SD rats (16 rats) with the low protein feed shown in Table 4, a catheter was inserted from the cervical vein under etherization, and was pushed until it reached the vicinity of the atrium, where the catheter was detained. After the operation, the rats were divided into four groups of A group (control group), B group, C group, and D group (the basic proteins-treated groups), and the rats in each group were treated with a control feed or feeds each containing the basic protein fraction-powder obtained in Example 3 at 0.1%, 1%, or 5% (Table 4).

**[Table 4]**

| | Low protein feed | Control group (A group) | Treated group (B group) | Treated group (C group) | Treated group (D group) |
|---|---|---|---|---|---|
| Casein | 4.0 | 10.0 | 9.9 | 9.0 | 5.0 |
| α-Cornstarch | 31.0 | 25.0 | 25.0 | 25.0 | 25.0 |
| Cellulose | 5.0 | 5.0 | 5.0 | 5.0 | 5.0 |
| Corn oil | 5.0 | 5. 0 | 5.0 | 5.0 | 5.0 |
| Sucrose | 50.2 | 50.2 | 50.2 | 50.2 | 50.2 |
| DL-Methionine | 0.3 | 0.3 | 0.3 | 0.3 | 0.3 |
| Vitamin mixture | 3.5 | 3.5 | 3.5 | 3.5 | 3.5 |
| Mineral mixture | 1.0 | 1.0 | 1.0 | 1.0 | 1.0 |
| Milk basic protein powder | - | - | 0.1 | 1.0 | 5.0 |

Four days after swiching of the feeds, blood collection was carried out with a catheter every 15 minutes to isolate serum. The growth hormone concentration in the obtained serum samples was measured by using a growth hormone ELISA kit (Rat/Mouse Growth Hormone ELISA Kit, Linco Research, Inc.).

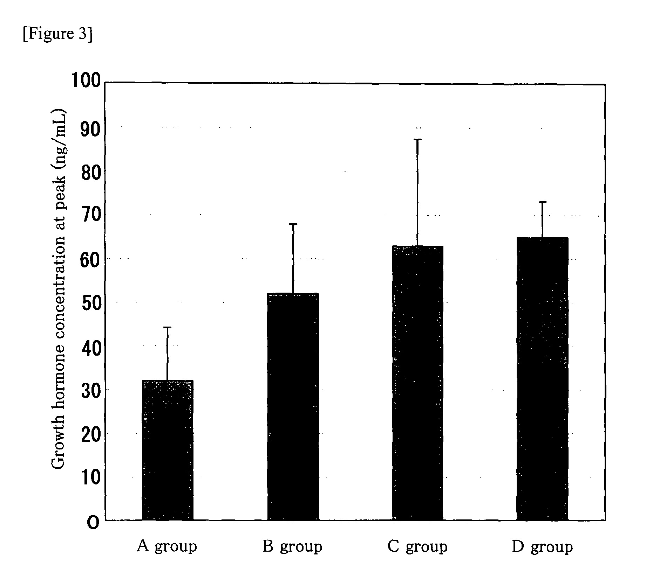

Figure 3 shows a change in the plasma growth hormone concentration of each experimental group. B group, C group, and D group, in which basic proteins were mixed, showed higher values in the growth hormone concentrations than A group. Thus, it was clarified that the basic protein fraction had the effect for promoting growth hormone-secretion.

### [Example 4]

The milk-derived basic protein fraction-powder obtained in Example 1 was used to prepare a tablet for promoting the growth hormone secretion having the composition shown in Table 5 by a conventional method.

**[Table 5]**

| | |
|---|---|
| Hydrous crystalline glucose | 73.5 (Weight %) |
| Soybean protein | 10 |
| Mineral mixture | 5 |
| Sugar ester | 1 |
| Flavor | 0.5 |
| Basic protein fraction-powder (Example 1) | 10 |

### EXAMPLE 5

The milk-derived basic protein fraction-powder obtained in Example 1 was used to prepare a drink having the composition shown in Table 6 and having a hormone-secretion promoting effect by a conventional method.

**[Table 6]**

| | |
|---|---|
| Mixed isomerized sugar | 15 (Weight %) |
| Fruit juice | 10 |
| Mineral mixture | 0.1 |
| Citric acid | 0.5 |
| Flavor | 0.1 |
| Water | 74.2 |
| Basic protein fraction-powder (Example 1) | 0.1 |

## Claims

1. An agent for promoting growth hormone-secretion, comprising a milk-derived basic protein fraction as an active ingredient.

2. An agent for promoting growth hormone-secretion according to claim 1, wherein the promotion of growth hormone-secretion is mediated by promotion of ghrelin secretion.

3. A method of promoting ghrelin secretion by using a milk-derived basic protein fraction.
